# EUROPEAN PATENT APPLICATION

(11) **EP 4 233 754 A1**
(43) Date of publication of application: **30.08.2023**
(21) Application number: 21910550.9
(22) Date of filing: 15.12.2021
(51) Int. Cl.: A61B 18/12, A61B 18/14

(54) **ELECTRODE UNIT AND HIGH FREQUENCY TREATMENT DEVICE**

(30) Priority: 25.12.2020 JP 2020217768
(71) Applicant: Kabushiki Kaisha Top, Adachi-ku, Tokyo 120-0035 (JP)
(72) Inventor: IKEUCHI, Atsushi, Tokyo 120-0035 (JP)
(74) Representative: Schön, Christoph
(86) International application number: PCT/JP2021/046384
(87) International publication number: WO 2022/138402

(57) **Abstract**

There is provided an electrode unit and a high-frequency treatment device capable of being appropriately used. In a high-frequency treatment device (1), an electrode unit (20) includes an electrode (21) disposed in a treatment target, a storage device (26) that stores information on the electrode (21), and a connector (24) that connects the electrode (21) and the storage device (26) to a high-frequency power generation device (10) that generates high-frequency power.

## Description

### Technical Field

The present invention relates to an electrode unit used in a high-frequency treatment and a high-frequency treatment device for performing a treatment such as thermal coagulation of nerve tissue or ablation of tumor tissue by applying a high-frequency current from an electrode disposed in a treatment target such as a human or an animal.

### Background Art

In the related art, in the medical field, a high-frequency treatment in which an electrode is disposed in a body of a human or an animal and a high-frequency current is passed from the electrode to perform ablation of a tissue or the like is widely used. Further, in recent years, as one of pain treatments, a method of performing nerve blocking by a high-frequency treatment has attracted attention (for example, refer to Patent Literature 1). The nerve blocking by the high-frequency treatment has advantages in that there are few side effects on surrounding tissues and the effect lasts for a long period, as compared with the method using a drug in the related art.

Generally, the electrode used for the high-frequency treatment is connected to a main body (high-frequency power generation device) that generates high-frequency power via a connector. That is, in a high-frequency treatment device that performs the high-frequency treatment, an electrode unit including the electrode and the connector connected to the electrode is a replacement component that is detachably connected to the main body. By doing so, it is possible not only to facilitate the replacement of the electrode according to occurrence of a defect, the number of times of use, or the like, but also to facilitate sterilization and disinfection of the electrode disposed in the body of the human, the animal, or the like. Therefore, it is possible to appropriately perform the high-frequency treatment.

### Citation List

### Patent Literature

Patent Literature 1: PCT Japanese Translation Patent Publication No. 2018-511444

### Summary of Invention

### Technical Problem

Meanwhile, in a case where the electrode unit is used as a replacement component, there is a problem in that an inappropriate electrode unit may be used such as, for example, a type that does not fit for the main body or a type that exceeds a use expiration date or a use limit. In addition, in a case where the inappropriate electrode unit is used, output control of the high-frequency power becomes unstable and the like, and not only the treatment is not performed appropriately, but also safety may be impaired.

In view of such circumstances, the present invention has an object to provide an electrode unit and a high-frequency treatment device capable of appropriately performing a high-frequency treatment.

### Solution to Problem

According to an aspect of the present invention, there is provided an electrode unit including: an electrode disposed in a treatment target; a storage device configured to store information on the electrode; and a connector configured to connect the electrode and the storage device to a high-frequency power generation device that generates high-frequency power.

In addition, according to another aspect of the present invention, there is provided a high-frequency treatment device including: the electrode unit according to the aspect of the present invention; and the high-frequency power generation device.

With the present invention, it is possible to determine whether or not the electrode unit connected to the high-frequency power generation device is appropriately usable, based on the information stored in the storage device, and thus it is possible to appropriately perform a high-frequency treatment.

Further, in the electrode unit according to the aspect of the present invention, the storage device may be disposed in the connector.

According to this, it is possible to dispose the storage device without significantly changing a shape of the electrode unit, so that it is possible to maintain usability of the electrode unit while providing the storage device.

Further, in the electrode unit according to the aspect of the present invention, the connector may have a contact terminal configured to be electrically connected to the high-frequency power generation device, and the storage device may be fixed to the connector by connecting a connection terminal of the storage device directly to the contact terminal.

According to this, it is possible to fix the storage device to the connector only by, for example, soldering without separately providing a support member or the like, so that it is possible to reduce a manufacturing cost.

Further, the electrode unit according to the aspect of the present invention, further includes: a temperature sensor configured to be connected to the high-frequency power generation device via the connector, in which the storage device may store information related to temperature measurement by the temperature sensor.

According to this, it is possible to cancel a measurement error caused by an individual difference of the temperature sensor included in the electrode unit connected to the high-frequency power generation device based on the information stored in the storage device, and thus it is possible to improve accuracy of the temperature measurement and appropriately perform the high-frequency treatment.

Further, the electrode unit according to the aspect of the present invention, further includes: a hollow hub configured to be connected to the electrode, in which the temperature sensor may be a thermocouple configured to be connected to the connector via a compensation lead wire, and the thermocouple and the compensation lead wire may be connected to each other via a bridge substrate disposed in the hub.

According to this, only by connecting end portions of the thermocouple and the compensation lead wire to a predetermined position on the bridge substrate by soldering or the like, it is possible to position and fix the end portions to be connected to each other, so that it is possible to achieve both reliable prevention of short-circuit of the thermocouple and facilitation of manufacture.

According to an electrode unit and a high-frequency treatment device according to the present invention, it is possible to obtain an excellent effect in that a high-frequency treatment is appropriately performed.

### Brief Description of Drawings

FIG. 1 is a schematic view illustrating an external appearance of a high-frequency treatment device according to an embodiment of the present invention.
FIG. 2 is a schematic diagram illustrating an external appearance of an electrode unit.
FIG. 3 is a schematic side cross-sectional diagram of an electrode and a hub.
FIG. 4 is a schematic side cross-sectional diagram of an electrode-side connector.

### Description of Embodiments

Hereinafter, embodiments of the present invention will be described with reference to the accompanying drawings.

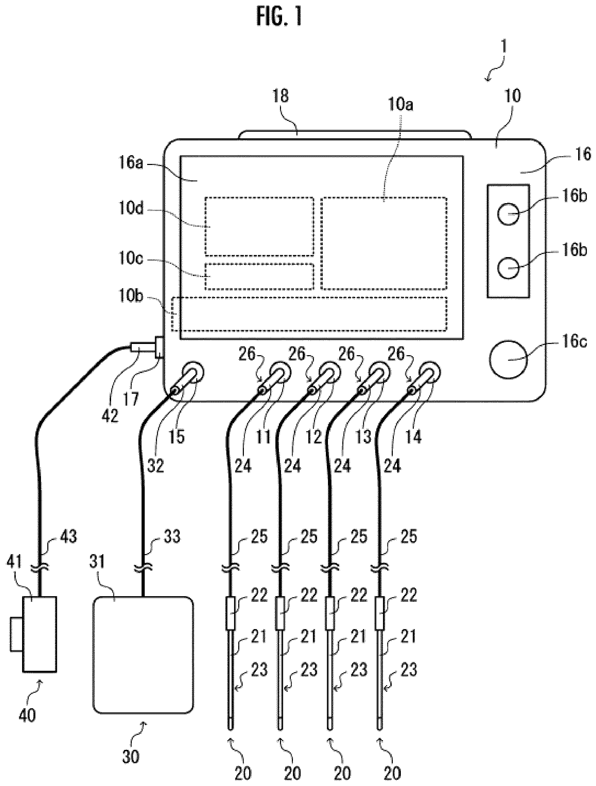

FIG. 1 is a schematic view illustrating an external appearance of a high-frequency treatment device 1 according to an embodiment of the present invention. The high-frequency treatment device 1 of the present embodiment is for performing nerve blocking by partially heating a peripheral nerve with a high-frequency current. As illustrated in FIG. 1, the high-frequency treatment device 1 includes a main body 10 which is a high-frequency power generation device, four electrode units 20 connected to the main body 10, a counter electrode plate unit 30 connected to the main body 10, and an operation switch unit 40 connected to the main body 10.

The main body 10 generates and outputs high-frequency power. On a front surface of the main body 10, four main body-side connectors 11 to 14 to which the electrode unit 20 is connected and a main body-side connector 15 to which the counter electrode plate unit 30 is connected are provided at a lower portion. An operation unit 16 that accepts an operation of a user is further provided on the front surface of the main body 10. The operation unit 16 is configured with a touch panel display 16a that accepts input operations such as various settings and displays various types of information, a button 16b that receives start and end operations of a treatment, and a control knob 16c for adjusting an output power.

A main body-side connector 17 to which the operation switch unit 40 is connected is provided on a left side surface of the main body 10. Further, a handle 18 for carrying the main body 10 is provided on an upper portion of the main body 10. Although not illustrated, a rear surface of the main body 10 is provided with a power connector and a power switch which are connected to a commercial alternating current power supply to receive a supply of power.

An output unit 10a, a switching unit 10b, a temperature measurement unit 10c, and a control unit 10d are provided inside the main body 10. The output unit 10a generates and outputs high-frequency power having a predetermined frequency (for example, 470 to 490 kHz) and a voltage (for example, 18 to 22 Vrms), based on power supplied from the commercial alternating current power supply. The output unit 10a is connected to the main body-side connectors 11 to 15 via the switching unit 10b.

The switching unit 10b switches a connection mode between the output unit 10a and the main body-side connectors 11 to 15. The temperature measurement unit 10c generates a temperature measurement signal (for example, 25 mV/°C) based on a signal (for example, voltage by thermoelectromotive power in the present embodiment) received from a temperature sensor (thermocouple 23 which will be described below in the present embodiment) included in the electrode unit 20, and transmits the temperature measurement signal to the control unit 10d. The control unit 10d controls each unit of the high-frequency treatment device 1 to execute a high-frequency treatment. Since a configuration of each unit of the main body 10 is a known configuration, detailed description thereof will be omitted.

The electrode unit 20 includes an electrode 21 that is to be inserted into a body of a patient as a treatment target to perform a high-frequency treatment, and is detachably connected to the main body 10. That is, in the electrode unit 20, the electrode 21 is replaceable with respect to the main body 10. Details of the electrode unit 20 will be described below.

The counter electrode plate unit 30 includes a counter electrode plate 31 that is a flat plate-shaped electrode attached and disposed on a skin surface of the patient as a treatment target, and is detachably connected to the main body 10. That is, the counter electrode plate 31 is replaceable with respect to the main body 10 in the same manner as the electrode 21. The counter electrode plate unit 30 includes a counter electrode plate 31, a counter electrode plate-side connector 32 connected to the main body-side connector 15, and a counter electrode plate cable 33 connecting the counter electrode plate 31 and the counter electrode plate-side connector 32.

The operation switch unit 40 is provided to the patient as a treatment target during a treatment, and is operated in a case where the patient feels pain. The operation switch unit 40 is configured with an operation switch 41 that is pressed by the patient, an operation switch-side connector 42 that is connected to a main body-side connector 17, and an operation switch cable 43 that connects the operation switch 41 and the operation switch-side connector 42.

During execution of the high-frequency treatment, the control unit 10d controls the output unit 10a to output high-frequency power, and controls the switching unit 10b to flow a high-frequency current between a plurality of electrodes 21 or between one or the plurality of electrodes 21 and the counter electrode plate 31. Accordingly, the high-frequency current flows in the body of the patient. The control unit 10d also controls the output of the high-frequency power so that a temperature measurement value based on the temperature measurement signal received from the temperature measurement unit 10c is substantially equal to a preset temperature setting value (control target value). Further, the control unit 10d causes the output unit 10a to reduce the output of the high-frequency power, based on the operation of the operation switch 41 during the treatment.

Next, the details of the electrode unit 20 will be described.

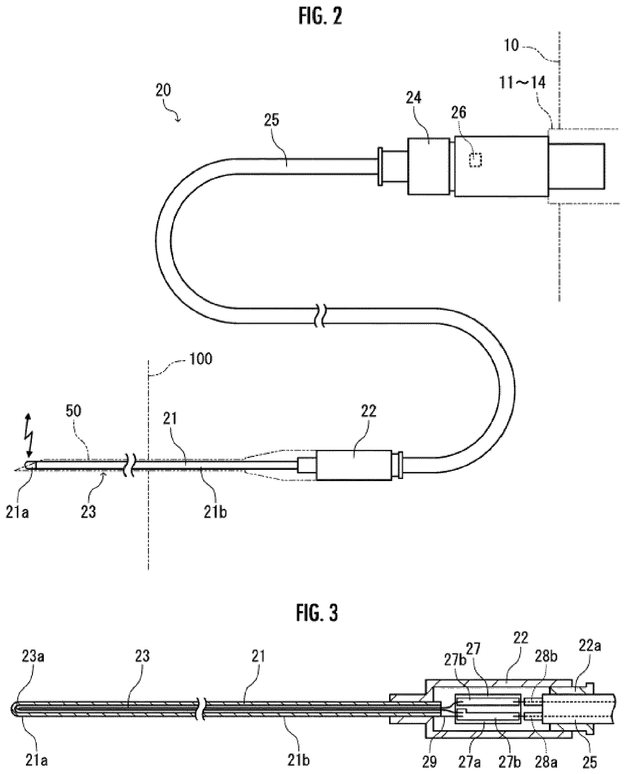

FIG. 2 is a schematic diagram illustrating an external appearance of the electrode unit 20. As illustrated in FIG. 2, the electrode unit 20 includes an electrode 21, a hub 22 connected to a rear end portion of the electrode 21, a thermocouple 23 (temperature sensor) disposed inside the electrode 21 and the hub 22, an electrode-side connector 24 connected to the main body-side connectors 11 to 14, and an electrode cable 25 connecting the hub 22 and the electrode-side connector 24. The electrode unit 20 also includes a storage device 26 disposed in the electrode-side connector 24.

The electrode 21 is an elongated tubular member made of a conductive metal. The electrode of the present embodiment is used by being inserted into a needle tube 50 pierced by a treatment target 100. Therefore, a tip of the electrode 21 is closed and rounded. In addition, the electrode 21 includes an insulating portion 21b in which most of the electrode 21 is coated in an insulating manner except for a non-insulating portion 21a at a tip portion. Therefore, the high-frequency current is output from the non-insulating portion 21a exposed at a tip portion of the needle tube 50.

FIG. 3 is a schematic side cross-sectional diagram of the electrode 21 and the hub 22. The hub 22 is a stepped cylindrical (hollow) member made of an appropriately insulating resin. The electrode 21 is inserted into a tip side of the hub 22 and adhered with an appropriate adhesive. Further, the electrode cable 25 is inserted together with the tubular lid member 22a into a rear end side of the hub 22, and is adhered with an appropriate adhesive. Therefore, an inside of the hub 22 is in a substantially sealed state.

The thermocouple 23 is disposed inside the electrode 21 along a longitudinal direction of the electrode 21, and a temperature measurement contact 23a is located inside the non-insulating portion 21a of the electrode 21. In the present embodiment, the thermocouple 23 and the electrode 21 are insulated by an insulating material filled in the electrode 21, and the temperature measurement contact 23a may be connected to the electrode 21. Further, in the present embodiment, the K-type (combination of chromel and alumel) thermocouple 23 is used, and needless to say, a type of thermocouple is not limited.

The thermocouple 23 is connected to a pair of compensation lead wires 28a and 28b via a bridge substrate 27 disposed inside the hub 22. The bridge substrate 27 is obtained by printing a circuit 27b on a surface of a substrate 27a made of an insulating material, and the thermocouple 23 and the compensation lead wires 28a and 28b are connected to the circuit 27b by soldering.

In the present embodiment, by connecting the thermocouple 23 and the compensation lead wires 28a and 28b via the bridge substrate 27 having a relatively high rigidity in this manner, both reliable short-circuit prevention and facilitation of manufacture are achieved. Specifically, it is possible to position and fix end portions of the thermocouple 23 and the compensation lead wires 28a and 28b, which tend to move due to bending deformation, on the bridge substrate 27, so that it is possible to reliably prevent a short-circuit of the thermocouple 23. Further, since it is possible to connect the thermocouple 23 and the compensation lead wires 28a and 28b to each other only by soldering the thermocouple 23 and the compensation lead wires 28a and 28b to predetermined positions on the bridge substrate 27, it is possible to facilitate manufacture.

Further, in the present embodiment, the bridge substrate 27 is fixed in the hub 22 by filling the inside of the hub 22 with an insulating resin, which also achieves facilitation of the manufacture and reduction of a cost. That is, a configuration of the hub 22 is simplified, and operations such as positioning of the bridge substrate 27 and engagement with the hub 22 at a time of manufacture are omitted, thereby facilitating the manufacture and reducing the cost.

In the present embodiment, further, by using the T-type (combination of copper and constantan) compensation lead wires 28a and 28b and also using the copper compensation lead wire 28a as a high-frequency current wire, facilitation of the manufacture and reduction of the cost are achieved. In the present embodiment, the electrode 21 and the circuit 27b of the bridge substrate 27 are connected via an intermediate wiring 29, and the rear end portion of the electrode 21 may be directly connected to the circuit 27b by soldering or the like.

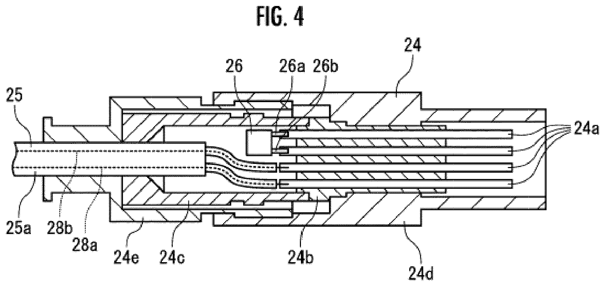

FIG. 4 is a schematic side cross-sectional diagram of the electrode-side connector 24. The electrode-side connector 24 includes four contact terminals 24a that are electrically connected to terminals of the main body-side connectors 11 to 14 of the main body 10, and an insulator 24b that supports these contact terminals 24a, and has a known configuration including a collet 24c, a shell 24d, and a back nut 24e. In the present embodiment, the contact terminal 24a of the electrode-side connector 24 is a male terminal, and needless to say, the contact terminal 24a may be a female terminal.

The electrode cable 25 includes a pair of coated compensation lead wires 28a and 28b, and a cover tube 25a that covers the compensation lead wires 28a and 28b. As described above, in the present embodiment, a configuration of the electrode cable 25 is simplified by using the copper compensation lead wire 28a also as a lead wire for a high-frequency current. The compensation lead wires 28a and 28b are respectively connected to the contact terminal 24a by soldering.

The storage device 26 stores information regarding the electrode 21 (electrode unit 20) and information regarding temperature measurement by the thermocouple 23 (temperature sensor). The storage device 26 is connected to the contact terminal 24a inside the electrode-side connector 24. Therefore, the control unit 10d of the main body 10 communicates with the storage device 26 via the main body-side connectors 11 to 14, and reads or writes the information.

In the present embodiment, by disposing the storage device 26 in the electrode-side connector 24, an empty space in the electrode-side connector 24 is effectively utilized. Further, by accommodating the storage device 26 in the electrode-side connector 24, it is possible to prevent a shape of the electrode unit 20 from being significantly changed as compared with a case where the storage device 26 is not provided. That is, it is possible to provide the storage device 26, without changing usability of the electrode unit 20.

The storage device 26 is configured with an electrically erasable programmable read only memory (EEPROM) including only two connection terminals of an input and output terminal 26a that also serves as a power supply terminal and a ground terminal 26b. As a result, in the present embodiment, it is possible to provide the storage device 26 while suppressing an increase in size of the electrode-side connector 24 by an increase in the number of contact terminals 24a.

Further, in the storage device 26, the input and output terminal 26a and the ground terminal 26b are directly connected to the contact terminal 24a by soldering. As a result, in the present embodiment, it is possible to fix the storage device 26 inside the electrode-side connector 24, without separately providing a support member or the like, thereby reducing the manufacturing cost.

The storage device 26 stores type information, manufacturing lot information, and use history information as the information regarding the electrode 21. Among these, the type information and the manufacturing lot information are stored in the storage device 26 at a time of manufacture of the electrode unit 20. In addition, the use history information is stored in the storage device 26 by the control unit 10d every time a high-frequency treatment is executed. The use history information includes information such as a date on which a treatment is executed, a treatment mode, and a treatment time.

The storage device 26 also stores correction information for correcting a temperature measurement signal (temperature measurement value), as the information regarding the temperature measurement by the thermocouple 23. This correction information is information for correcting a measurement error caused by an individual difference of the thermocouple 23, the compensation lead wires 28a and 28b, and the like. The electrode unit 20 individually measures a measurement error in temperature measurement at a time of manufacture, and stores the correction information based on the measurement error in the storage device 26.

When executing the high-frequency treatment, the control unit 10d first accesses the storage device 26 of the electrode unit 20 connected to the main body-side connectors 11 to 14, and acquires the type information, the manufacturing lot information, the use history information, and the correction information. Based on the type information, the manufacturing lot information, and the use history information, it is determined whether or not the electrode unit 20 is a genuine product, whether or not a type is appropriate for the main body 10, and whether or not a use expiration date or a use limit is exceeded, and the like.

That is, the control unit 10d determines whether or not the electrode 21 (electrode unit 20) is appropriately usable, based on the information regarding the electrode 21 acquired from the storage device 26. The control unit 10d executes the high-frequency treatment only in a case where it is determined that the electrode 21 is appropriately usable. In a case where the control unit 10d also determines that the electrode 21 is not appropriately usable, the control unit 10d displays an error display on the touch panel display 16a, and does not execute the high-frequency treatment.

In addition, during execution of the high-frequency treatment, the control unit 10d corrects the temperature measurement signal received from the temperature measurement unit 10c based on the correction information, and calculates a temperature measurement value. Since the correction information cancels a measurement error caused by the individual difference of the thermocouple 23 or the like, the temperature measurement value becomes accurate, and thus the output control of the high-frequency power is performed with high accuracy. In particular, even in a case where the electrode unit 20 is replaced, the control unit 10d acquires new correction information from the storage device 26 of the replaced electrode unit 20 to perform correction, and thus it is possible to perform the output control with high accuracy without requiring an input operation or the like of the correction information.

The control unit 10d also stores the use history information in the storage device 26 when the high-frequency treatment is ended. By referring to the use history information stored in this manner, it is possible not only to determine a use limit (life) of the electrode 21, but also to investigate the cause in a case where a defect occurs in the electrode 21.

In this manner, since the electrode unit 20 of the present embodiment includes the storage device 26, the control unit 10d of the main body 10 refers to information on the electrode 21 stored in the storage device 26, and thus it is possible to determine whether or not the electrode 21 (electrode unit 20) is appropriately usable. In addition, the control unit 10d is capable of correcting the temperature measurement value during the treatment and performing the output control with high accuracy, by using the information regarding the temperature measurement by the thermocouple 23 stored in the storage device 26. That is, with the electrode unit 20 of the present embodiment, it is possible to appropriately perform the high-frequency treatment.

The electrode 21 may be configured to have a blade surface at the tip portion, and be able to be pierced into the human body or the like. Further, the electrode 21 may be configured to have a hole at the tip portion, and to deliver a chemical solution or the like. In addition, for example, a temperature sensor other than the thermocouple 23, such as a temperature measurement resistor, may be disposed inside the electrode 21, or a temperature sensor separate from the electrode 21 may be provided and disposed in the vicinity of the electrode 21.

Further, the electrode cable 25 may be provided with a lead wire for a high-frequency current separately from the compensation lead wires 28a and 28b. In addition, the storage device 26 may be disposed on an outer surface of the electrode-side connector 24, or may be disposed in a location other than the electrode-side connector 24, such as inside the hub 22 or inside the cover tube 25a of the electrode cable 25, for example. Further, the storage device 26 may be configured with a memory other than the EEPROM, and needless to say, the number of terminals included in the storage device 26 and a type of interface are not particularly limited.

Further, the storage device 26 and the contact terminal 24a may be connected by a method other than soldering, for example, crimping or the like, and an appropriate connector, socket, or the like may be interposed between the storage device 26 and the contact terminal 24a. In the same manner, the compensation lead wires 28a and 28b may be connected to the bridge substrate 27 or the contact terminal 24a by crimping or the like.

Although the embodiments of the present invention are described above, the electrode unit and the high-frequency treatment device according to the present invention are not limited to the embodiments described above, and are variously modified without departing from the gist of the present invention.

For example, the shape and arrangement of each unit of the high-frequency treatment device 1 are not limited to the shapes and arrangements illustrated in the embodiments described above, and it is possible to adopt various known shapes and arrangements. Further, the high-frequency treatment device 1 is not limited to the device for nerve blocking, and may be used for other purposes such as ablation of a tumor, for example.

Further, the electrode 21 is not limited to an electrode to be inserted into the inside of the human body or the like, and may be, for example, an electrode that is disposed on the skin surface of the human body and heats a relatively shallow region under the skin. In addition, the number of electrode units 20 that are connectable to the main body 10 is not particularly limited. Further, the operation switch unit 40 and the process related to the operation switch unit 40 may be omitted.

In addition, the actions and effects illustrated in the embodiments described above are merely a list of the most appropriate actions and effects resulting from the present invention, and the actions and effects according to the present invention are not limited thereto.

### Description of Reference Numerals

1 high-frequency treatment device
10 main body (high-frequency power generation device)
20 electrode unit
21 electrode
22 hub
23 thermocouple (temperature sensor)
24 electrode-side connector (connector)
24a contact terminal
26 storage device
26a input and output terminal (connection terminal)
26b ground terminal (connection terminal)
27 bridge substrate
28a, 28b compensation lead wire

## Claims

1. An electrode unit comprising:
an electrode disposed in a treatment target;
a storage device configured to store information of the electrode; and
a connector configured to connect the electrode and the storage device to a high-frequency power generation device that generates high-frequency power.

2. The electrode unit according to claim 1,
wherein the storage device is disposed in the connector.

3. The electrode unit according to claim 2,
wherein the connector has a contact terminal configured to be electrically connected to the high-frequency power generation device, and
the storage device is fixed to the connector by connecting a connection terminal of the storage device directly to the contact terminal.

4. The electrode unit according to any one of claims 1 to 3, further comprising:
a temperature sensor configured to be connected to the high-frequency power generation device via the connector,
wherein the storage device stores information related to temperature measurement by the temperature sensor.

5. The electrode unit according to claim 4, further comprising:
a hollow hub configured to be connected to the electrode,
wherein the temperature sensor is a thermocouple configured to be connected to the connector via a compensation lead wire, and
the thermocouple and the compensation lead wire are connected to each other via a bridge substrate disposed in the hub.

6. A high-frequency treatment device comprising:
the electrode unit according to any one of claims 1 to 5; and
a high-frequency power generation device.
